# EUROPEAN PATENT APPLICATION

(11) **EP 3 378 454 A1**
(43) Date of publication of application: **26.09.2018**
(21) Application number: 15908744.4
(22) Date of filing: 18.11.2015
(51) Int. Cl.: A61F 13/496

(54) **CLOTHING ARTICLE**

(71) Applicant: Unicharm Corporation, Shikokuchuo-shi, Ehime 799-0111 (JP)
(72) Inventor: KATSURAGAWA, Kunihiko, Kanonji-shi Kagawa 769-1602 (JP); TAKINO, Syunsuke, Kanonji-shi Kagawa 769-1602 (JP); UEDA, Masumi, Kanonji-shi Kagawa 769-1602 (JP)
(74) Representative: Staeger & Sperling Partnerschaftsgesellschaft mbB
(86) International application number: PCT/JP2015/082382
(87) International publication number: WO 2017/085804

(57) **Abstract**

There is provided a diaper (1) including: a front exterior member (2a), a back exterior member (2b), a plurality of elastic members (4), and a plurality of cuts (5). Each of the front exterior member (2a) and the back exterior member (2b) includes a non-skin-side sheet (22) and a skin-side sheet (21). Between the non-skin-side sheet (22) and the skin-side sheet (21), the plurality of elastic members (4) are provided in a longitudinal direction. The plurality of cuts (5) are provided in at least either one of the non-skin-side sheet (22) and the skin-side sheet (21) of at least either one of the front exterior member (2a) and the back exterior member (2b). A first cut (51) and a second cut (52) are provided between a first elastic member (41) and a second elastic member (42) adjacent to each other. A first overlapping part (5a) is a part in which the first cut (51) and the second cut (52) overlap in a lateral direction. A third cut (53) and a fourth cut (54) are provided between the second elastic member (42) and a third elastic member (43). A second overlapping part (5b) is a part in which the third cut (53) and the fourth cut (54) overlap in the lateral direction. The first overlapping part (5a) and the second overlapping part (5b) overlap in the lateral direction.

## Description

### [Technical Field]

The present invention relates to a wearable article.

### [Background Art]

As a conventional wearable article, a pull-on disposable diaper and the like have been known. For example, PTL 1 discloses a wearable article including: an exterior member formed into pants shape having a waist opening edge and a pair of leg opening edges; and waist elastic members extending along the width direction and arranged in a waist part between the waist opening edge and the leg opening edges.

The exterior member includes: an inner sheet arranged on the skin side of a wearer; an outer sheet arranged on the non-skin side; and an intermediate sheet arranged therebetween. The outer sheet and the intermediate sheet are joined, on the waist part, by a plurality of joined sections lined in the width direction. The waist elastic members are arranged between the inner sheet and the intermediate sheet, and are joined, in a region having the joined sections, to at least either one of the inner sheet and the intermediate sheet. When the waist elastic members contract in the width direction, regions between pairs of adjacent joined sections project on the outer sheet from the non-skin side, forming regular creases (pleats) extending along the longitudinal direction.

### [Citation List]

### [Patent Literature]

[PTL 1] Japanese Patent Application Publication No. 2015-104605

### [Summary of Invention]

### [Technical Problem]

In such a diaper disclosed in PTL 1, the outer sheet and the intermediate sheet are joined on the plurality of joined sections, and thereby regions extending along the longitudinal direction and having high rigidities are provided, forming regular, remarkable creases. However, when a wearer put on the diaper, the joined sections having higher rigidity (harder) than materials of the sheet members come into contact with the wearer's skin, making the wearer discomfort.

In view of the problem described above, it is an advantage of the present invention to provide a wearable article which can suppress wearer's discomfort on his/her skin and makes creases more visually recognizable.

### [Solution to Problem]

An aspect of the present invention to achieve the above advantage is a wearable article having a longitudinal direction, a lateral direction, and a thickness direction that intersect one another, the wearable article including: a front exterior member arranged along the lateral direction; a back exterior member arranged along the lateral direction; a plurality of elastic members extending along the lateral direction; and a plurality of cuts having a predetermined lateral length. Each of the front exterior member and the back exterior member includes a non-skin-side sheet located on a non-skin side and a skin-side sheet located on a skin side. The plurality of elastic members are provided lined in the longitudinal direction between the non-skin-side sheet and the skin-side sheet. The plurality of cuts are disposed of at least either one of the non-skin-side sheet and the skin-side sheet of at least either one of the front exterior member and the back exterior member. A first cut and a second cut of the plurality of cuts are provided between a first elastic member and a second elastic member adjacent in the longitudinal direction of the plurality of elastic members, the first cut being located closer to the first elastic member than to the second elastic member, the second cut being located closer to the second elastic member than to the first elastic member. A first overlapping part is a part in which the first cut and the second cut overlap in the lateral direction. A third cut and a fourth cut of the plurality of cuts are provided between the second elastic member and a third elastic member adjacent in the longitudinal direction of the plurality of elastic members, the third cut being located closer to the second elastic member than to the third elastic member, the fourth cut being located closer to the third elastic member than to the second elastic member. A second overlapping part is a part in which the third cut and the fourth cut overlap in the lateral direction. The first overlapping part and the second overlapping part overlap in the lateral direction.

Other features of this invention will become apparent from the description in this specification and the attached drawings.

### [Advantageous Effects of Invention]

According to the present invention, it is possible to suppress wearer's discomfort on his/her skin and makes creases more visually recognizable.

### [Brief Description of Drawings]

FIG. 1 is a schematic perspective view of an example of the configuration of a diaper according to an embodiment of the present invention.
FIG. 2 is a schematic plan view of the diaper 1 with being spread out, as viewed from the skin side.
FIG. 3A is a diagram illustrating a positional relation of a plurality of cuts in a skin-side sheet and a non-skin-side sheet. FIG. 3B is a schematic lateral view of FIG. 3A.
FIG. 4A is a schematic view, in the thickness direction, of a skin-side sheet and a non-skin-side sheet with elastic members contracting in the lateral direction. FIG. 4B is a schematic view, in the longitudinal direction, of a skin-side sheet and a non-skin-side sheet with an elastic member contracting in the lateral direction.

### [Description of Embodiments]

At least the following matters will be made clear by the description in the present specification and the accompanying drawings.

A wearable article having a longitudinal direction, a lateral direction, and a thickness direction that intersect one another,
the wearable article including:
a front exterior member arranged along the lateral direction;
a back exterior member arranged along the lateral direction;
a plurality of elastic members extending along the lateral direction; and
a plurality of cuts having a predetermined lateral length,
   each of the front exterior member and the back exterior member including a non-skin-side sheet located on a non-skin side and a skin-side sheet located on a skin side,
   the plurality of elastic members being provided lined in the longitudinal direction between the non-skin-side sheet and the skin-side sheet,
   the plurality of cuts disposed of at least either one of the non-skin-side sheet and the skin-side sheet of at least either one of the front exterior member and the back exterior member,
   a first cut and a second cut of the plurality of cuts being provided between a first elastic member and a second elastic member adjacent in the longitudinal direction of the plurality of elastic members,
      the first cut being located closer to the first elastic member than to the second elastic member,
      the second cut being located closer to the second elastic member than to the first elastic member,
   a first overlapping part being a part in which the first cut and the second cut overlap in the lateral direction,
   a third cut and a fourth cut of the plurality of cuts being provided between the second elastic member and a third elastic member adjacent in the longitudinal direction of the plurality of elastic members,
      the third cut being located closer to the second elastic member than to the third elastic member,
      the fourth cut being located closer to the third elastic member than to the second elastic member,
   a second overlapping part being a part in which the third cut and the fourth cut overlap in the lateral direction,
   the first overlapping part and the second overlapping part overlapping in the lateral direction.

With such a wearable article, each cut exists at a longitudinal position close to one of the elastic members. Accordingly, the elastic member contracts in the lateral direction, fine creases are produced between the elastic member and the cut, and the creases are finer than creases produced in regions located both lateral sides of the region in which the cut is provided. Consequently, the creases become more likely to be recognized. In addition, lining the first to fourth cuts in the longitudinal direction makes the creases easier to be neatly formed in the region in which the cut is provided. And this decreases rigidity. Accordingly, it is possible to suppress wearer's discomfort on his/her skin.

In such a wearable article, it is preferable
that a plurality of the first overlapping parts are arranged at a lateral interval by lining at a lateral interval a plurality of pairs of the first cut and the second cut,
that a plurality of the second overlapping parts are arranged at a lateral interval by lining at a lateral interval a plurality of pairs of the third cut and the fourth cut, and
that each of the plurality of first overlapping parts overlaps in the lateral direction a corresponding one of the plurality of second overlapping parts.

With such a wearable article, regions each having the first to fourth cuts which are lined in the longitudinal direction are lined at intervals in the lateral direction. Accordingly, when the elastic member contract in the lateral direction, a plurality of regular creases are formed, making the creases more visually recognizable.

In such a wearable article, it is preferable that, concerning at least one of a set of the first cuts, a set of the second cuts, a set of the third cuts, and a set of the fourth cuts, a distance between laterally adjacent cuts of at least the set is greater than a lateral length of a cut of at least the set.

With such a wearable article, regions in which no cut is provided are arranged between regions in which laterally adjacent cuts are provided. This prevents the cuts from being torn apart by a force applied when a wearer puts on the wearable article and pulls it up from his/her legs toward his/her crotch, making it possible to maintain the strengths of the front exterior member and the back exterior member.

In such a wearable article, it is preferable that, concerning at least one of a set of the first cuts, a set of the second cuts, a set of the third cuts, and a set of the fourth cuts, a distance between a central cut and a left cut that are adjacent in the lateral direction is different from a distance between the central cut and a right cut that are adjacent in the lateral direction.

With such a wearable article, the intervals of the cuts lined in the lateral direction are not uniform. This increases the probability that the cuts are located on parts projecting in the thickness direction (creases) by contraction of the elastic member, and this makes it possible to improve visibility of the cut.

In such a wearable article, it is preferable
that a lateral length of the second cut is equal to a lateral length of the third cut, and
that the second cut and the third cut overlap throughout from a lateral one end to a lateral other end.

With such a wearable article, when the second elastic member contracts, a crease produced between the second elastic member and the second cut matches in the lateral direction with a crease produced between the second elastic member and the third cut. This increases lateral regions of the creases, making it possible for the creases to become more visually recognizable.

In such a wearable article, it is preferable
that a distance between the second cut and the third cut is smaller than a distance between the first cut and the second cut, and
that the distance between the second cut and the third cut is smaller than a distance between the third cut and the fourth cut.

With such a wearable article, since a cut is located at a position closer to an elastic member, a region between the elastic member and the cut becomes narrower. Consequently, a force of the elastic member is more likely to be exerted on the region, producing more fine creases and making the creases more remarkable.

In such a wearable article, it is preferable
that a longitudinal distance between the first cut and the second cut is greater than a lateral length of the first cut and a lateral length of the second cut, and
that a longitudinal distance between the third cut and the fourth cut is greater than a lateral length of the third cut and a lateral length of the fourth cut.

With such a wearable article, a region longitudinally sandwiched between the first cut and the second cut, and a region longitudinally sandwiched between the third cut and the fourth cut have rectangular shapes whose longitudinal sides are longer. Accordingly, lateral contraction of the elastic members cause the entirety of the region to project in the thickness direction, making it easier to produce creases extending along the longitudinal direction.

In such a wearable article, it is preferable that the wearable article further comprises a band-shaped crotch member including a one-end portion arranged on the front exterior member and another-end portion arranged on the back exterior member, that the plurality of cuts are arranged on both lateral sides of each of the one-end portion and the other-end portion, and that the plurality of cuts are not arranged on a side longitudinally upper than the one-end portion and the other-end portion.

With such a wearable article, the plurality of cuts are arranged on the lateral sides of the crotch member, which tends to be stuffy. This makes it easier for humid air to go out through the cuts. In addition, no cut is arranged in a part upper than the crotch member, which is pulled by a wearer at the time of putting on the wearable article, making it possible to maintain the strength of the part.

In such a wearable article, it is preferable that the cut is line-shaped along the lateral direction.

With such a wearable article, the cut is line-shaped along the lateral direction. This makes it easier for the cut to open longitudinally when the elastic member contracts in the lateral direction, improving visibility of the cut.

In such a wearable article, it is preferable that the cut extends through the skin-side sheet and the non-skin-side sheet in the thickness direction.

With such a wearable article, the cut extends through the skin-side sheet and the non-skin-side sheet in the thickness direction. This makes it possible to visually recognize wearer's skin through the cut, further improving visibility of the cut.

In such a wearable article, it is preferable
that at least either one of the front exterior member and the back exterior member includes
   a part projecting on the skin side by contraction of the plurality of elastic members in the lateral direction and
   a part projecting on the non-skin side by contraction of the plurality of elastic members in the lateral direction, and
that the first cut, the second cut, the third cut, and the fourth cut are arranged in the part projecting on the non-skin side.

With such a wearable article, at least either one of the front exterior member and the back exterior member includes a part projecting on the non-skin side when the plurality of elastic members contract in the lateral direction, and the first to fourth cuts are arranged in the projecting part. Accordingly, the openings of the first to fourth cuts becomes more likely to be recognized from outside, and this makes it possible to improve visibility of the first to fourth cuts.

In such a wearable article, it is preferable that at least either one of the skin-side sheet and the non-skin-side sheet is a colored sheet.

With such a wearable article, using a colored sheet makes shadow more distinguishable, making it further easier to visually recognize creases and the cuts.

### = Embodiment =

A disposable diaper 1 (hereinafter merely referred to as a diaper 1) is described as an example of a wearable article according to an embodiment of the present invention.

### < Configuration of Diaper 1 >

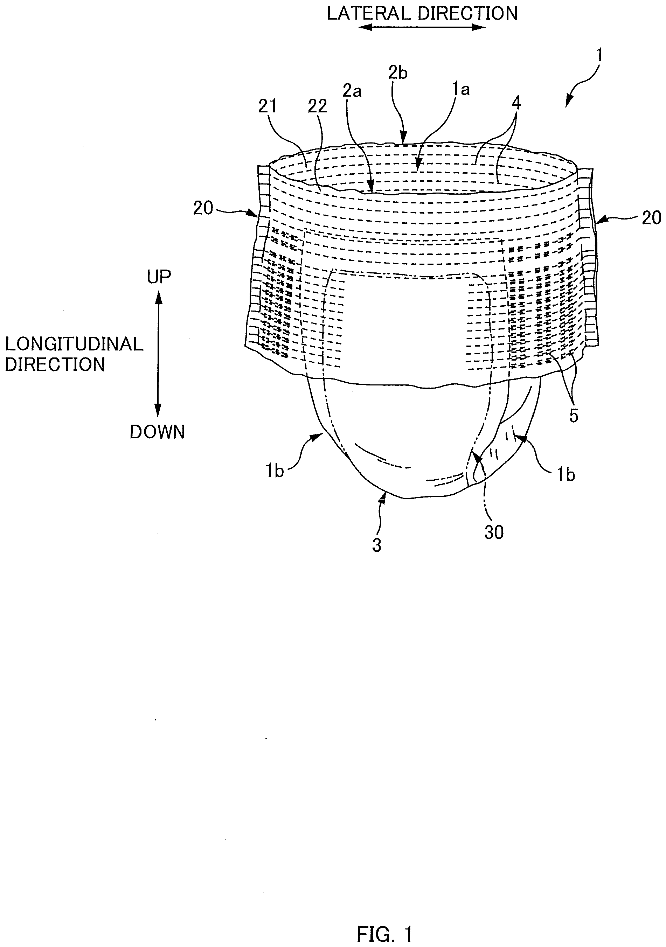

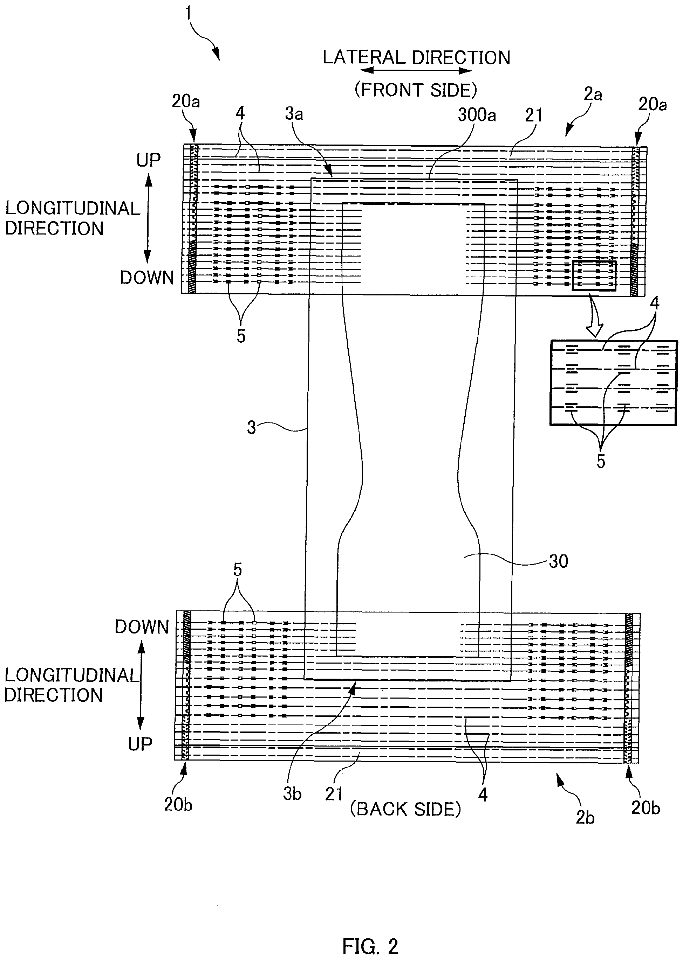

First, the configuration of the diaper 1 is described with reference to FIGS. 1 and 2.

FIG. 1 is a schematic perspective view of an example of the configuration of a diaper 1 according to an embodiment. FIG. 2 is a schematic plan view of the diaper 1 with being spread out, as viewed from the skin side.

The diaper 1 has the longitudinal direction, the lateral direction and the thickness direction which intersect one another. In the longitudinal direction, the waist side of a wearer is defined as the "up" side, and the crotch side of the wearer is defined as the "down" side. In the thickness direction, a side which comes into contact with wearer's skin is defined as a "skin side", and the opposite side is defined as a "non-skin side".

The diaper 1 according to the present embodiment is, as shown in FIG. 1, a so-called 3-piece diaper including: a front exterior member 2a arranged on the wearer's front side; a back exterior member 2b arranged on the wearer's back side; and a band-shaped crotch member 3. As shown in FIG. 2, a lengthwise one-end portion 3a of the crotch member 3 is arranged on the front exterior member 2a, and a lengthwise other-end portion 3b is arranged on the back exterior member 2b. And, the crotch member 3 includes an absorbent body 30 therein, which absorbs liquid such as urine. In FIG. 1, the absorbent body 30 is indicated by double-dotted chain lines.

When the diaper 1 is spread out, the front exterior member 2a and the back exterior member 2b are rectangular as viewed from above, and have lateral long sides. The diaper 1 is folded along substantially a lengthwise center of the crotch member 3 so that the front exterior member 2a faces the back exterior member 2b, and then joining lateral ends 20a of the front exterior member 2a and lateral ends 20b of the back exterior member 2b. Thereby, as shown in FIG. 1, joined portions 20 are formed in lateral ends of the diaper 1 which is worn, to form an annular waist opening 1a and a pair of leg openings 1b.

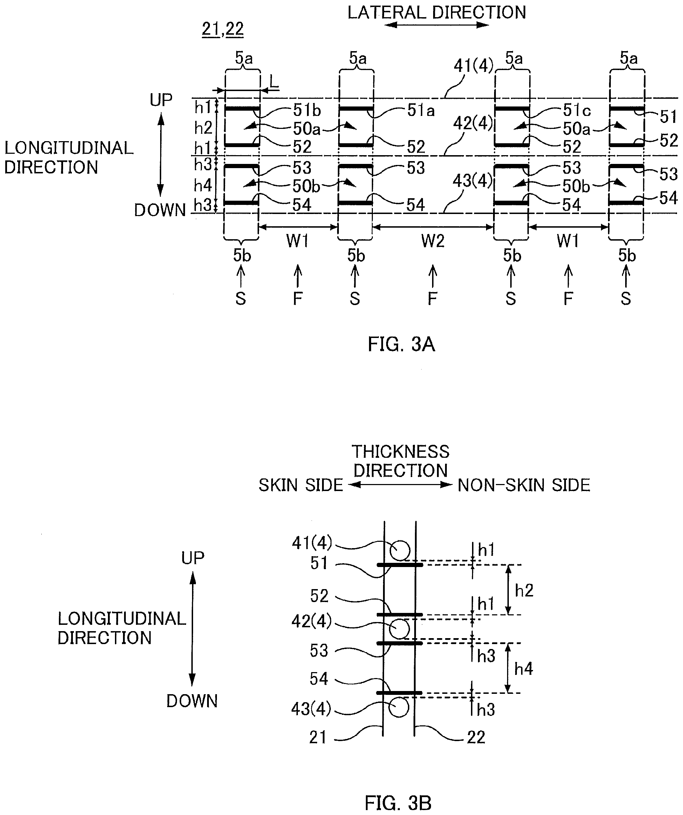

Each of the front exterior member 2a and the back exterior member 2b includes a skin-side sheet 21 on the skin side and a non-skin-side sheet 22 on the non-skin side (see FIGS. 1 and 3B). Each of the skin-side sheet 21 and the non-skin-side sheet 22 has a rectangular shape whose long side is in the lateral direction as viewed from above, and is formed of a flexible member such as nonwoven fabric. In the present embodiment, at least either one of the skin-side sheet 21 and the non-skin-side sheet 22 is a colored sheet. However, it is not essential to use a colored sheet.

Between the skin-side sheet 21 and the non-skin-side sheet 22, a plurality of elastic members 4 extending along the lateral direction are provided lined in the longitudinal direction. In the present embodiment, each elastic member 4 is an elastic string whose cross section is circular (see FIG. 3C). But, It is not essential that the elastic member 4 is an elastic string, and it is sufficient that the elastic member is capable of stretching in the lateral direction. The plurality of elastic members 4 are indicated by dashed lines in FIG. 1, and by single-dotted chained lines in FIG. 2.

In the present embodiment, in each of the front exterior member 2a and the back exterior member 2b, a plurality of cuts 5 are provided on both of the skin-side sheet 21 and the non-skin-side sheet 22, and the plurality of cuts 5 has a predetermined lateral length. In both of the front exterior member 2a and the back exterior member 2b, it is not necessary for the plurality of cuts 5 to be provided on both of the skin-side sheet 21 and the non-skin-side sheet 22. That is, it is sufficient that at least either one of the front exterior member 2a and the back exterior member 2b has the plurality of cuts 5 formed on at least either one of its own skin-side sheet 21 and its own non-skin-side sheet 22.

As shown in FIG. 2, the plurality of cuts 5 in the front exterior member 2a are arranged on both lateral sides of the lengthwise one-end portion 3a of the crotch member 3, but are not arranged on a side longitudinally upper than the lengthwise one-end portion 3a. The description "a side longitudinally upper than the lengthwise one-end portion 3a" means a region upper in the longitudinal direction of the front exterior member 2a than a position of the edge 300a of the lengthwise one-end portion 3a, more specifically, a region longitudinally upper than the longitudinal position where the edge 300a and virtual lines laterally extending from the edge 300a are located.

Thus, the plurality of cuts 5 are arranged on the lateral sides of the crotch member 3, which tends to be stuffy when a wearer puts on a diaper 1. Accordingly, a larger amount of air passes through the plurality of cuts 5, making it possible to improve air permeability. A part upper in the longitudinal direction of the diaper 1 than the lengthwise one-end portion 3a of the crotch member 3 is a part held by a wearer, at the time of putting on the diaper 1, in order to pull up the diaper 1 toward his/her waist. Since no cut 5 are provided in the part, the strength of the part can be maintained. This can prevent the front exterior member 2a from being torn apart when a wearer pulls up the diaper 1.

In the present embodiment, as shown in FIG. 2, in the back exterior member 2b, the plurality of cuts 5 are arranged on the lateral sides of the lengthwise other-end portion 3b of the crotch member 3. And, the plurality of cuts 5 are arranged in the longitudinal direction upper than the lengthwise other-end portion 3b. However, it is not essential that the plurality of cuts 5 are arranged in the longitudinal direction upper than the lengthwise other-end portion 3b. Also, it is not essential that the plurality of cuts 5 are arranged in a configuration shown in FIG. 2.

As shown in a partial magnified view of FIG. 2, each of the plurality of cuts 5 are arranged at a position close to an elastic member 4. On both the lateral sides of at least the lengthwise one-end portion 3a and lengthwise other-end portion 3b of the crotch member 3, the plurality of elastic members 4 are arranged more densely compared to on a side longitudinally upper than the lengthwise one-end portion 3a and the lengthwise other-end portion 3b. Accordingly, when a diaper 1 is put on, the plurality of cuts 5 are arranged densely on wearer's leg sides. This decreases the rigidities of the skin-side exterior member 2a and the back exterior member 2b in portions around legs, improving flexibility and air permeability. This makes it possible to suppress the stuffiness and tightness of the portions around legs. It is not essential that the plurality of cuts 5 are arranged densely on wearer's leg sides.

### < Relation between Elastic Members 4 and Cuts 5 >

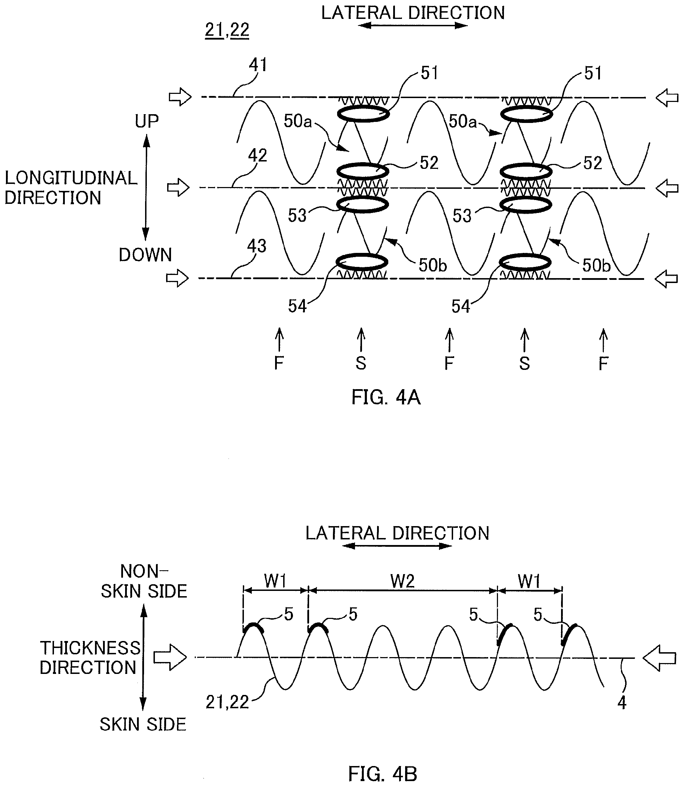

Next, a relation between the elastic members 4 the cuts 5 is described with reference to FIGS. 3A, 3B, 4A and 4B.

FIG. 3A is a diagram illustrating a positional relation of the plurality of cuts 5 in the skin-side sheet 21 and the non-skin-side sheet 22. FIG. 3B is a schematic lateral view of FIG. 3A. FIG. 4A is a schematic view, in the thickness direction, of the skin-side sheet 21 and the non-skin-side sheet 22 with the elastic members 4 contracting in the lateral direction. FIG. 4B is a schematic view, in the longitudinal direction, of the skin-side sheet 21 and the non-skin-side sheet 22 with the elastic member 4 contracting in the lateral direction.

Among the plurality of elastic members 4, a first elastic member 41, a second elastic member 42, and a third elastic member 43 are described as examples. Among the plurality of cuts 5, a first cut 51, a second cut 52, a third cut 53, and a fourth cut 54 are described as examples.

Among the plurality of elastic members 4, concerning the first elastic member 41 and the second elastic member 42 adjacent in the longitudinal direction, the first cut 51 and the second cut 52 are provided between these elastic members. The first cut 51 is located at a position closer to the first elastic member 41 than to the second elastic member 42, and the second cut 52 is located at a position closer to the second elastic member 42 than to the first elastic member 41. That is, the first cut 51 is located at a position closer to the first elastic member 41, and the second cut 52 is located at a position closer to the second elastic member 42.

As shown in FIG. 3A, in the present embodiment, the distance h1 between the first cut 51 and the first elastic member 41 is smaller than the distance h2 between the first cut 51 and the second cut 52, and also the distance h1 between the second cut 51 and the second elastic member 42 is smaller than the distance h2 (h1 < h2). Accordingly, the first cut 51 is located at a position closer to the first elastic member 41, and the second cut 52 is located at a position closer to the second elastic member 42.

As shown in FIG. 3A, the first cut 51 and the second cut 52 have a first overlapping part 5a in which these cuts overlap in the lateral direction. In the present embodiment, the lateral length of the first cut 51 is equal to the lateral length of the second cut 52, and the first overlapping part 5a extends from one ends to the other ends of the first cut 51 and the second cut 52 throughout in the lateral direction. It is not necessary for the first overlapping part 5a to overlap from one ends to the other ends of the first cut 51 and the second cut 52 throughout in the lateral direction.

Among the plurality of elastic members 4, concerning the second elastic member 42 and the third elastic member 43 adjacent in the longitudinal direction, the third cut 53 and the fourth cut 54 are provided between these elastic members. The third cut 53 is located at a position closer to the third elastic member 43 than to a fourth elastic member 44, and the fourth cut 54 is located at a position closer to the fourth elastic member 44 than to the third elastic member 43. That is, the third cut 53 is located at a position closer to the second elastic member 42, and the fourth cut 54 is located at a position closer to the third elastic member 43. The second cut 52 and the third cut 53 are arranged so that the second elastic member 42 is sandwiched therebetween in an up-down direction (the longitudinal direction).

As shown in FIG. 3A, in the present embodiment, the distance h3 between the third cut 53 and the second elastic member 42 is smaller than the distance h4 between the third cut 53 and the fourth cut 54, and also the distance h3 between the fourth cut 54 and the third elastic member 43 is smaller than the distance h4 (h3 < h4). Accordingly, the third cut 53 is located at a position closer to the second elastic member 42, and the fourth cut 54 is located at a position closer to the third elastic member 42.

The following distances are equal: the distance h1 between the first cut 51 and the first elastic member 41; the distance h1 between second cut 51 and the second elastic member 42; the distance h3 between the third cut 53 and the second elastic member 42; and the distance h3 between the fourth cut 54 and the third elastic member 43 (h1 = h3). In addition, the distance h2 between the first cut 51 and the second cut 52 is equal to the distance h4 between the third cut 53 and the fourth cut 54 (h2 = h4). The distance (h1 + h3) between the second cut 52 and the third cut 53 is smaller than the distance h2 between the first cut 51 and the second cut 52, and also is smaller than the distance h4 between the first cut 51 and the second cut 52 (h1 + h3 < h2 = h4).

It is not essential that the distances h1, h2, h3 and h4 have the foregoing relations. It is sufficient that the first cut 51 and the second cut 52 are located between the first elastic member 41 and the second elastic member 42, and that the third cut 53 and the fourth cut 54 are located between the second elastic member 42 and the third elastic member 43.

Similarly to the first cut 51 and the second cut 52, the third cut 53 and the fourth cut 54 have a second overlapping part 5b in which these cuts overlap in the lateral direction. In the present embodiment, the lateral length of the third cut 53 is equal to the lateral length of the fourth cut 54, and therefore, the second overlapping part 5b extends from one ends to the other ends of the third cut 53 and the fourth cut 54 throughout in the lateral direction. Similarly to the first overlapping part 5a, it is not necessary for the second overlapping part 5b to overlap from one ends to the other ends of the third cut 53 and the fourth cut 54 throughout in the lateral direction.

The first overlapping part 5a and the second overlapping part 5b overlap in the lateral direction, and the first cut 51, the second cut 52, the third cut 53, and the fourth cut 54 are arranged lined in the longitudinal direction. Here, a region extending along the longitudinal direction and having the first to fourth cuts 51 to 54 is defined as a region S.

In the present embodiment, the lateral length of the second cut 52 is equal to the lateral length of the third cut 53, and therefore the first to fourth cuts 51 to 54 have an identical lateral length (indicated by symbol L in FIG. 3A). The second cut 52 and the third cut 53 overlap throughout from the lateral one end to the lateral other end, and therefore the first overlapping part 5a and the second overlapping part 5b overlap throughout in the lateral direction. Accordingly, the first to fourth cuts 51 to 54 are aligned in the longitudinal direction, the region S is a region having a rectangular shape whose longitudinal sides are longer.

It is not essential for the first cut 51, the second cut 52, the third cut 53, and the fourth cut 54 to be aligned in the longitudinal direction. As long as at least the first overlapping part 5a and the second overlapping part 5b overlap in the lateral direction, it is acceptable that the first to fourth cuts 51 to 54 does not match in the lateral direction.

In the skin-side exterior member 2a and the non-skin-side exterior member 2b, as shown in FIGS. 4A and 4B, when the first elastic member 41, the second elastic member 42, and the third elastic member 43 each contract in the lateral direction, the skin-side sheet 21 and the non-skin-side sheet 22 contract in the lateral direction, forming a part projecting in the thickness direction, that is, a crease.

Specifically speaking, the skin-side exterior member 2a and the non-skin-side exterior member 2b include parts projecting on the skin side in the thickness direction (creases projecting from the skin side) and parts projecting on the non-skin side (creases projecting from the non-skin side), and these projecting parts are formed by lateral contractions of the first elastic member 41, the second elastic member 42, and the third elastic member 43. As shown in FIG. 4B, the parts projecting on the skin side and the parts projecting on the non-skin side are more likely to be formed lined alternately in the lateral direction.

Here, as shown in FIG. 4A, the first to fourth cuts 51 to 54 each open by getting both lateral ends closer to each other and widening longitudinally the central part. In the present embodiment, since each of the first to fourth cuts 51 to 54 is line-shaped along the lateral direction as shown in FIG. 3A, the cut is easy to widen longitudinally to open, improving its visibility.

As shown in FIG. 3B, the first to fourth cuts 51 to 54 (cuts 5) extend through the skin-side sheet 21 and the non-skin-side sheet 22 in the thickness direction. This make it possible to visually recognize the wearer's skin through openings of the first to fourth cuts 51 to 54 (cuts 5), further improving the visibility of the cuts 5.

It is not essential that each of the first to fourth cuts 51 to 54 (cuts 5) is line-shaped along the lateral direction. Neither, It is not essential that the cuts 51 to 54 (cuts 5) extend through the skin-side sheet 21 and the non-skin-side sheet 22 in the thickness direction.

Low rigidities of the skin-side sheet 21 and the non-skin-side sheet 22 around the openings makes it easier to produce fine creases in the following areas: an area between the first elastic member 41 and the first cut 51; an area between the second elastic member 42 and the second cut 52; an area between the second elastic member 42 and the second cut 52; and an area between the third elastic member 43 and the fourth cut 54.

The first cut 51 is located at a position closer to the first elastic member 41. And, the second cut 52 and the third cut 53 are located at a position closer to the second elastic member 42, and the fourth cut 54 is located at a position closer to the third elastic member 43. Accordingly, the following regions become narrower: The longitudinal region between the first cut 51 and the first elastic member 41; the longitudinal region between the second cut 52 and the second elastic member 42; the longitudinal region between the third cut 53 and the second elastic member 42; and the longitudinal region between the fourth cut 54 and the third elastic member 43. Consequently, forces of the first elastic member 41, the second elastic member 42, and the third elastic member 43 are more likely to be exerted on the respective regions, producing more fine creases and making the creases more remarkable.

In the region S, a plurality of regions in which fine creases are produced are lined in the longitudinal direction, and therefore creases extending along the longitudinal direction are neatly formed, making it possible for the creases to become more visually recognizable. As mentioned above, in the present embodiment, since at least either one of the skin-side sheet 21 and the non-skin-side sheet 22 is a colored sheet, shadow becomes more distinguishable, making it further easier to visually recognize creases and the first to fourth cuts 51 to 54 (cuts 5). In order to make shadow more distinguishable, it is desirable that the color of the colored sheet is dark.

Next to each of the lateral sides of each region S, there are a region F (regions in which the first to fourth cuts 51 to 54 are not provided) . Providing the first to fourth cuts 51 to 54 to the region S makes the rigidity of the region S smaller than the rigidity of the regions F. Accordingly, it is possible to suppress wearer's discomfort on his/her skin.

In the present embodiment, a crease formed between the second elastic member 42 and the second cut 52 matches in the lateral direction with a crease formed between the second elastic member 42 and the third cut 53. That is, those creases formed facing each other across the second elastic member 42 in the longitudinal direction match in the lateral direction. This increases the lateral region of an overlapping part in which two creases formed near the second elastic member 42 overlap, making it possible for the creases to become more visually recognizable.

The longitudinal distance h2 between the first cut 51 and the second cut 52 is greater than the lateral length L of the first cut 51 (the second cut 52, the third cut 53, and the fourth cut 54), and the longitudinal distance h4 between the third cut 53 and the fourth cut 54 is greater than the lateral length L of the first cut 51 (the second cut 52, the third cut 53, and the fourth cut 54) (h2 = h4 > L).

In the present embodiment, the longitudinal distance h2 between the first cut 51 and the second cut 52 is equal to the longitudinal distance h4 between the third cut 53 and the fourth cut 54 (h2 = h4 > L). However, it is sufficient that at least the followings are satisfied: the longitudinal distance h2 between the first cut 51 and the second cut is greater than the lateral length L of the first cut 51 (the second cut 52) (h2 > L); and the longitudinal distance h4 between the third cut 53 and the fourth cut 54 is greater than the lateral length L of the third cut 53 (the fourth cut 54) (h4 > L) .

Thus, a region 50a longitudinally sandwiched between the first cut 51 and the second cut 52, and a region 50b longitudinally sandwiched between the third cut 53 and the fourth cut 54 have rectangular shapes whose longitudinal sides are longer. Accordingly, the lateral contractions of the first elastic member 41, the second elastic member 42, and the third elastic member 43 cause the entireties of the regions 50a and 50b to project in the thickness direction, making it easier to produce creases extending along the longitudinal direction.

In the present embodiment, the distance h2 between the first cut 51 and the second cut 52 is equal to the distance h4 between the third cut 53 and the fourth cut 54 (h2 = h4). Accordingly, the region 50a longitudinally sandwiched between the first cut 51 and the second cut 52 is equal to the region 50b longitudinally sandwiched between the third cut 53 and the fourth cut 54. However, it is not essential that these regions are equal.

Next, a relation of the lateral positions of the first to fourth cuts 51 to 54 will be described below. As shown in FIG. 3A, in a diaper 1, a plurality of pairs of the first cut 51 and the second cut 52 are placed at lateral intervals, and thus a plurality of the first overlapping parts 5a are arranged at the lateral intervals. And, a plurality of pairs of the third cut 53 and the fourth cut 54 are placed at lateral intervals, and thus a plurality of the second overlapping parts 5b are arranged at the lateral intervals. Each of the plurality of first overlapping parts 5a overlaps in the lateral direction corresponding one of the plurality of second overlapping parts 5b. Accordingly, the regions S and the regions F are arranged alternately lined in the lateral direction. Thus, the regions S are lined at intervals in the lateral direction, and thereby a plurality of creases are formed in a laterally regular manner, making it possible for the creases to become more visually recognizable.

The plurality of cuts 5 are composed of a plurality of groups whose minimum unit is a set of the first to fourth cuts 51 to 54, the groups lined in the longitudinal direction and in the lateral direction.

In FIG. 3A, among the plurality of first cuts 51 lined in the lateral direction, the first cut 51 located leftmost is defined as a first left cut 51b, the first cut 51 located second from the left is defined as a first central cut 51a, and the first cut 51 located second from the right is defined as a first right cut 51c. The plurality of the first cuts 51 are arranged so that the distance W1 between the first central cut 51a and the first left cut 51b that are adjacent in the lateral direction is different from the distance W2 between the first central cut 51a and the first right cut 51c that are adjacent in the lateral direction.

In the present embodiment, the distance W1 between the first central cut 51a and the first left cut 51b is smaller than the distance W2 between the first central cut 51a and the first right cut 51c (W1 < W2). But, It is not essential that W1 < W2, and it is sufficient that W1 ≠ W2.

Assuming that the lateral contraction of the elastic members 4 causes the lateral contractions of the skin-side sheet 21 and the non-skin-side sheet 22, and projections in the thickness direction are made on the skin side or on the non-skin side. In this case, if the plurality of first cuts 51 lined in the lateral direction at a regular interval are located on parts not projecting in the thickness direction, the plurality of first cuts 51 are located between the creases, becoming less likely to be recognized from outside.

However, if the intervals of the plurality of first cuts 51 lined in the lateral direction are not uniform, the probability increases that the first cuts 51 are located on parts projecting in the thickness direction (creases), as shown in FIG. 4B. This makes it possible to improve visibility of the plurality of first cuts 51. The same is applied to the plurality of second cuts 52, the plurality of third cuts 53, and the plurality of fourth cuts 54.

In order to improve visibility of the first to fourth cuts 51 to 54 by making the openings of the cuts 51 to 54 easier to be recognized from outside, it is preferable that, as shown in FIG. 4B, the first to fourth cuts 51 to 54 are arranged in a part projecting on the non-skin side on each of the front exterior member 2a and the non-skin-side exterior member 2b.

For each of the first to fourth cuts 51 to 54, distances W1 and W2 between cuts adjacent in the lateral direction are longer than the lateral length L of each of the first to fourth cuts 51 to 54 (W1 > L and W2 > L). That is, the lateral width of the region F is greater than the lateral width of the region S.

As mentioned above, the regions F in which the first to fourth cuts 51 to 54 are not provided have a lateral width greater than the regions S in which the first to fourth cuts 51 to 54 are provided. This prevents the first to fourth cuts 51 to 54 from being torn apart by a force applied when a wearer puts on the diaper 1 and pulls it up from his/her legs toward his/her crotch. This makes it possible to maintain the strengths of the front sheet 21 and the back sheet 22, that is, the strengths of the front exterior member 2a and the back exterior member 2b.

### = Other Embodiments =

The above-mentioned embodiment is provided for facilitating the understanding of the present invention, and is not to be interpreted as limiting the present invention. The present invention can be altered and improved without departing from the gist thereof and the invention includes equivalent thereof. For example, the present invention can be altered as described below.

In the foregoing embodiment, a so-called three-piece diaper consisting of three components a front exterior member 2a, a back exterior member 2b and a crotch member 3 is described. However, it is not necessary for the present invention to be limited to a three-piece diaper. It is not essential that a wearable article is a diaper.

### [Reference Signs List]

1 diaper (wearable article)
2a front exterior member
2b back exterior member
3 crotch member
3a lengthwise one-end portion
3b lengthwise other-end portion
4 elastic member
5 cut
5a, 5b first and second overlapping parts
21 skin-side sheet
22 non-skin-side sheet
41 to 43 first to third elastic member
51 to 54 first to fourth cuts
51a first central cut (central cut)
51b first left cut (left cut)
51c first right cut (right cut)

## Claims

1. A wearable article having a longitudinal direction, a lateral direction, and a thickness direction that intersect one another,
the wearable article comprising:
a front exterior member arranged along the lateral direction;
a back exterior member arranged along the lateral direction;
a plurality of elastic members extending along the lateral direction; and
a plurality of cuts having a predetermined lateral length,
each of the front exterior member and the back exterior member including a non-skin-side sheet located on a non-skin side and a skin-side sheet located on a skin side,
the plurality of elastic members being provided lined in the longitudinal direction between the non-skin-side sheet and the skin-side sheet,
the plurality of cuts disposed of at least either one of the non-skin-side sheet and the skin-side sheet of at least either one of the front exterior member and the back exterior member,
a first cut and a second cut of the plurality of cuts being provided between a first elastic member and a second elastic member adjacent in the longitudinal direction of the plurality of elastic members,
the first cut being located closer to the first elastic member than to the second elastic member,
the second cut being located closer to the second elastic member than to the first elastic member,
a first overlapping part being a part in which the first cut and the second cut overlap in the lateral direction,
a third cut and a fourth cut of the plurality of cuts being provided between the second elastic member and a third elastic member adjacent in the longitudinal direction of the plurality of elastic members,
the third cut being located closer to the second elastic member than to the third elastic member,
the fourth cut being located closer to the third elastic member than to the second elastic member,
a second overlapping part being a part in which the third cut and the fourth cut overlap in the lateral direction,
the first overlapping part and the second overlapping part overlapping in the lateral direction.

2. A wearable article according to claim 1, wherein
a plurality of the first overlapping parts are arranged at a lateral interval by lining at a lateral interval a plurality of pairs of the first cut and the second cut,
a plurality of the second overlapping parts are arranged at a lateral interval by lining at a lateral interval a plurality of pairs of the third cut and the fourth cut, and
each of the plurality of first overlapping parts overlaps in the lateral direction a corresponding one of the plurality of second overlapping parts.

3. A wearable article according to claim 2, wherein
concerning at least one of a set of the first cuts, a set of the second cuts, a set of the third cuts, and a set of the fourth cuts,
a distance between laterally adjacent cuts of at least the set is greater than a lateral length of a cut of at least the set.

4. A wearable article according to claim 2 or 3, wherein
concerning at least one of a set of the first cuts, a set of the second cuts, a set of the third cuts, and a set of the fourth cuts,
a distance between a central cut and a left cut that are adjacent in the lateral direction is different from a distance between the central cut and a right cut that are adjacent in the lateral direction.

5. A wearable article according to any one of claims 1 to 4, wherein
a lateral length of the second cut is equal to a lateral length of the third cut, and
the second cut and the third cut overlap throughout from a lateral one end to a lateral other end.

6. A wearable article according to any one of claims 1 to 5, wherein
a distance between the second cut and the third cut is smaller than a distance between the first cut and the second cut, and
the distance between the second cut and the third cut is smaller than a distance between the third cut and the fourth cut.

7. A wearable article according to any one of claims 1 to 6, wherein
a longitudinal distance between the first cut and the second cut is greater than a lateral length of the first cut and a lateral length of the second cut,
a longitudinal distance between the third cut and the fourth cut is greater than a lateral length of the third cut and a lateral length of the fourth cut.

8. A wearable article according to any one of claims 1 to 7, wherein
the wearable article further comprises a band-shaped crotch member including
a one-end portion arranged on the front exterior member and another-end portion arranged on the back exterior member,
the plurality of cuts are arranged on both lateral sides of each of the one-end portion and the other-end portion, and
the plurality of cuts are not arranged on a side longitudinally upper than the one-end portion and the other-end portion.

9. A wearable article according to any one of claims 1 to 8, wherein
the cut is line-shaped along the lateral direction.

10. A wearable article according to any one of claims 1 to 9, wherein
the cut extends through the skin-side sheet and the non-skin-side sheet in the thickness direction.

11. A wearable article according to any one of claims 1 to 10, wherein
at least either one of the front exterior member and the back exterior member includes
a part projecting on the skin side by contraction of the plurality of elastic members in the lateral direction and
a part projecting on the non-skin side by contraction of the plurality of elastic members in the lateral direction, and
the first cut, the second cut, the third cut, and the fourth cut are arranged in the part projecting on the non-skin side.

12. A wearable article according to any one of claims 1 to 11, wherein
at least either one of the skin-side sheet and the non-skin-side sheet is a colored sheet.
